# EUROPEAN PATENT SPECIFICATION

(11) **EP 0 591 197 B1**
(45) Date of publication and mention of the grant of the patent: **26.04.2000**
(21) Application number: 91916262.8
(22) Date of filing: 16.08.1991
(51) Int. Cl.: A61F 11/00, A61F 11/08

(54) **HEARING PROTECTIVE EARPLUG HAVING ALTERNATIVE MODES OF INSERTION**
GEHÖRSCHUTZOHRSTÖPSEL MIT ALTERNATIVEN EINSETZWEISEN
BOUCHON DE PROTECTION AURICULAIRE PRESENTANT DES MODES D'INSERTION ALTERNATIFS

(30) Priority: 20.08.1990 US 570348
(43) Date of publication of application: 13.04.1994
(73) Proprietor: Cabot Safety Intermediate Corporation, Boston, MA 02108 (US)
(72) Inventor: GARDNER, Ross, Jr., Indianapolis, IN 46268 (US)
(74) Representative: Attfield, Donald James
(86) International application number: US9105928
(87) International publication number: WO9203112

(56) References cited:
- WO-A-92/00049
- DE-A- 3 536 793
- FR-A- 1 395 197
- GB-A- 733 542
- GB-A- 2 203 349
- LU-A- 58 560
- US-A- 3 881 570
- US-A- 3 895 627
- US-A- 4 122 841
- US-A- 4 158 087
- US-A- 4 253 452
- US-E- R E29 487

## Description

### BACKGROUND OF THE INVENTION

The present invention relates generally to hearing protective earplugs and is more particularly concerned with premolded, polymeric foam earplugs of the push-in type.

Premolded polymeric foam earplugs composed of resilient polymer foam materials such as foam rubber, polyurethane or plasticized polyvinylchloride are well known in the art. For instance, in the United Kingdom Patent No. 733,542, to Hultgren, there is disclosed a push-in type earplug comprising a soft, elastic, bullet-shaped spongy body element having a stiff axially oriented stem by which to facilitate insertion and removal of the spongy body element into and from the ear canal. Hultgren also discloses the possibility of controlling the acoustic attenuation properties of his plug by varying the pore size and density of the spongy body element. A major problem generally incurred with premolded earplugs of the prior art resides in the anatomical fact that the human ear can is quite variable in size and geometry and, as a result, a single size of a premolded prior art earplug has not been found capable of accommodating the broad range of human ear canal sizes. Thus, premolded earplugs are generally produced in several sizes which, of course, increases the complexity and cost of manufacture and is bothersome throughout the distribution chain in terms of expense and complexity of purchasing and inventory control. As in the case of many other prior art premolded plugs, the earplugs of Hultgren are also said to be produced in two or three sizes. In addition, in order to assure good attenuation performance, the Hultgren plugs, as in the case of any earplug which is provided in several sizes, should be fitted to the individual wearer by a skilled hearing protection specialist. Unlike my present invention, the bullet-shaped spongy body element of the earplug disclosed by Hultgren is not possessed of viscoelastic recovery properties.

United States Patent No. 2,438,339, issued to M. J. Thomas, discloses a frusto-conically shaped earplug of the push-in type having an elongated stiff cylindrical core composed of a material such as hard rubber. This core is surrounded by a frusto-conically shaped body composed of soft expandable and contractible material such as sponge rubber. The embedded stiff core is disclosed to be coextensive with the frusto-conically shaped body; that is to say, it does not extend outside the body element and thus cannot serve as a stem by which to manipulate the plug into and from the ear canal. Rather, the sole function served by the core element in the Thomas invention is that of stiffening of the body element such that the foam body is prevented from excessive lateral bending or distortion during insertion of the plug into the ear canal. In addition, Thomas mentions nothing with respect to the recovery rate or other properties of the body element of her earplug construction.

In West German OS 2 325 823, to Envac Establishment, filed on May 22, 1973 and laid open on December 19, 1974, there is disclosed an earplug comprising a spherical polymeric foam body element having an essentially impermeable outer surface and a stiff elongate handle extending therefrom. The foam body element can be composed of such polymeric materials as polyurethane or plasticized polyvinylchloride. As in the Hultgren and Thomas patents discussed above, this opened West German application neither discloses nor suggests a viscoelastic foam body element and the plug construction taught therein is solely of the push-in type.

In my prior U.S. Patent Nos. 3,811,437 and Re. 29,487 there are taught certain roll-down type hearing protective earplugs composed of viscoelastic polymeric foam and having a size and shape adapted to be compressed, inserted into the ear canal and therein allowed to expand to result in a comfortable and complete obturation of the ear canal. Earplugs manufactured in accordance with the aforementioned patents have met with outstanding commercial success in the marketplace due to their features of easy insertability, comfort, excellent attentuation properties and their ability to be produced in a single size while competently fitting almost the entire adult population. Nevertheless, the foam earplugs do possess certain deficiencies which mitigate against their use in certain hearing protective situations. Firstly, the earplugs of my above-identified patents are prepared for insertion by initially rolling them down between thumb and fingers, thereby to compress them to below the sizes of the ear canals into which they are to be inserted. In terms of hygiene, therefore, the user's hands should be at least relatively clean at the time of use. This is not a trivial matter because in many noisy industrial environments there are abrasive materials or harsh chemicals which can become imbedded in the earplug. These contaminants may be present on the workers' hands at the time of use of the plugs and the necessity for first cleansing the hands can be a bothersome requisite in such situations. Additionally, while the preliminary step of rolling the plugs between the fingers is, indeed, a simple physical step, hand disabled users, such as those suffering from arthritis, can find the roll-down step a near or actual impossibility.

In International patent publication WO-A-9200049 there is described a single flange earplug comprising a rounded nose end, an integral rearwardly directed skirt defining a flange and a rearwardly directed axial stem extending from the said nose.

### OBJECTS OF THE INVENTION

It is a principal object of the invention to provide a new and novel hearing protective earplug construction.

It is still another object of the invention to provide a new and novel earplug construction which may be utilized either as a push-in or roll-down plug.

It is another object of the invention to provide an earplug construction which may be produced in a single size, but which provides easy insertion, wearer comfort and good sound attenuation to substantially the entire adult population.

Other objects and advantages of the invention will in part be obvious and will in part appear hereinafter.

### SUMMARY OF THE INVENTION

The earplug of the invention comprises a soft, resilient polymeric foam body comprising a smoothly contoured viscoelastic main body element having a rounded nose end and a maximum cross sectional dimension at any point along its length of 13.73 mm (0.540 inch), said foam body comprising a base end opposite said nose end of the main body element. An elongate stem having two ends, one end being embedded and secured to the interior of the nose end portion of the main body element said stem extending axially and rearwardly from the nose end portion and through the base end and the other end of said stem terminating at a point exterior said base end, said stem being sufficiently stiff to allow manipulation of said main body element into an ear canal whether in a precompressed or uncompressed condition, said viscoelastic main body element of the earplug having a 90% recovery rate, as determined by the test described hereinafter, of between 2 and 120 seconds.

The main body element may have an outboard end which transitions into a terminal flared end portion having a maximum cross sectional dimension which is sufficiently large to enable the terminal flared end portion to function as a stop means on insertion of the earplug into the ear canal.

The earplug of the invention being an improvement over that of the nearest prior art in that the foam body is homogeneous, being free of lateral projections and internal cavities other than a stem securing recess, and the base end extending linearly across the maximum dimension of the body element.

### BRIEF DESCRIPTION OF THE DRAWINGS

**Figure 1** hereof is a side view, partly in section, of an earplug in accordance with the invention,
**Figure 2** is a side view, partly in section, of another embodiment of an earplug in accordance with the invention.

### DESCRIPTION OF THE PREFERRED EMBODIMENTS

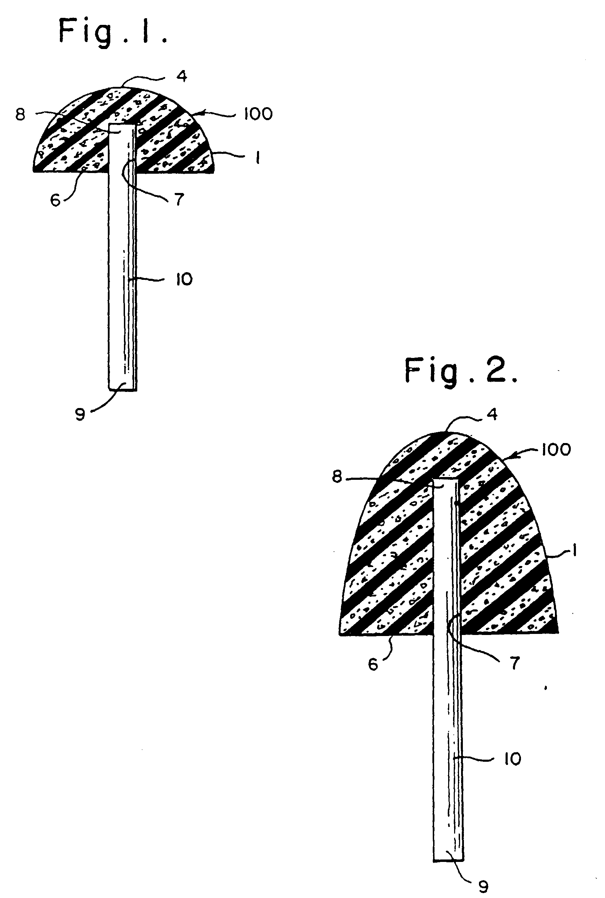

Referring now to Figures 1 and 2, wherein like reference numerals refer to like structures, the earplug construction of the invention broadly comprises a soft, resilient polymeric foam body 100 comprising a smoothly contoured, homogeneous, viscoelastic main body element 1 adapted in size and shape to be inserted into the human ear canal in acoustically obturating relationship therewith and an elongate stem 10 extending rearwardly and axially therefrom. The main body element 1 is, for purposes of the invention, defined as that portion of the polymeric foam body 100 which is insertable into the ear canal. While the preferred cross sectional shape of the main body element 1 is circular, it will be appreciated that said cross sectional shape may also be ovoid or elliptical. By the term "homogeneous" it is meant that the main body element 1 of the body 100 is devoid of lateral projections or internal cavities, such as in the nature of one or more flange elements or substantial cavities therein (other than the stem-receiving recess 7). In order to achieve the essentially universal fit benefit of the invention and depending somewhat upon the density and softness of the polymeric foam of which it is composed and the specific geometry of the foam main body element 1, the maximum cross sectional dimensions thereof at any point along its length can be up to about 13.72mm (0.540 inch).

The main body element 1 may transition into a terminal flared end portion lying at the outboard end and which flared end portion has a larger maximum cross sectional dimension than that of the external auditory meatus or opening of the average human ear canal. Where this design consideration is met the flared end portion functions as a convenient stop means whereby an appropriate depth of insertion of the main body element 1 into the ear canal is facilitated. Upon insertion of the main body element 1 into the ear canal, using the roll-down or push-in mode of insertion, the terminal flared end portion ultimately butts against the opening of the ear canal, thereby to signal the user that the appropriate insertion depth has been attained. Additionally, upon said butting of the terminal flared end portion against the opening of the ear canal, over-insertion of the main body element 1 is rendered difficult due to the interference provided by said flared end portion.

Stem 10 is an elongate member which may be tubular or solid throughout its cross section and may be uniform or slightly tapered along its length. In the case of a slightly tapered configuration, the larger end will normally be utilized as the end 8 inserted into recess 7 of the foam main body element 1. In any event the cross sectional dimension of stem 10 at said end 8 should not be so great as to prevent easy insertion of the earplug into ear canals of small size. To this end I prefer that the maximum cross sectional dimension of the stem 10 at end 8 be within the range of from 3.2mm (0.125 inch) to 4.0 mm (0.16 inch). The stem 10, of course, can be of any convenient length such that the free end 9 thereof extends beyond the body element 1 so as to provide a conveniently manipulable insertion and removal member for said foam main body element 1. Thus the exact length selected for the stem 10 will largely be a matter of choice. Of course the dimensions, geometry and material of construction employed for the stem 10 are selected such that the stem 10 will be sufficiently stiff as to allow manipulation of the main foam body element 1 into the ear canal whether in the precompressed or uncompressed condition.

Many stem 10 constructions will suggest themselves to those of skill in the art and are suitable. For instance paper, rubber, cardboard and plastic rod forms of the type often used as medical cotton swab holders are generally of adequately stiff nature and appropriate cross sectional dimension for use in the present earplug construction. So, too, are many known plastic drinking and stirring straws. Whatever the selection of the stem 10 material, however, it is important that the end 8 thereof be attached to the foam main body element 1 with sufficient security as to avoid separation of the stem 10 from the main body element 1 during use. The security of attachment can be achieved in any suitable manner, such as by use of suitable adhesives or by solvent or thermal welding. In a preferred embodiment of the invention the foam body element 100 is composed of a molded polyurethane foam and the stem 10 is affixed to the main body element 1 thereof during the molding operation. For example, the stem 10 can be utilized as an insert in the mold for the foam body 100, the end 8 thereof acting as a male old member for recess 7. As the foam formulation blows and cures in the mold to form the foam body element 100 and the main body element 1 thereof, it additionally bonds firmly and tenaciously to the end 8 of the stem 10.

The smoothly contoured main body element 1 extends rearwardly from a rounded nose or forward end 4. The length of the main body element 1 relative to its maximum cross sectional dimension is subject to considerable variation and is not normally critical. For example, shape of body element 1 of the earplug shown in Figure 1 is in the nature of a hemisphere; therefore, its length to diameter ratio is about 0.5 to 1. Contrastingly, in the embodiment of Figure 2, the body element 1 is shown to be distinctly bullet shaped and has a length to diameter ratio of about 3 to 2. Where the overall body comprises a terminal flared portion the length of the main body element 1 is taken from the nose end thereof to the point whereat transition thereof into the terminal flared end portion begins. Thus the length to diameter ratio is similar to that of the embodiment of Figure 2, in other words about 3 to 2.

The relative hardness value of a small ware composed of soft resilient polymeric foam is generally difficult to measure with precision by the Shore OO Durometer technique. However, with the sole intention of providing a general guideline and with no intention whatsoever of limiting the invention, I have so far found that instantaneous Shore OO Durometer values of acceptable foams for use as the body element 1 in the present invention appear to be no greater than about 60 Duometer units and have usually been found to reside within the range of from about 20 to about 45 Shore OO Durometer units. By "instantaneous" value it is meant that the reading of the Duometer is taken immediately after application of the indentor foot load to the sample and without affording the foam sample time to substantially creep or relax under the load of the indentor foot subsequent to its application.

In general I prefer that the apparent density of the viscoelastic polymeric foam material of construction of the main body element 1 be no greater than about 0.32 g/cm³ (20lbs/ft³) and, even more preferably, no greater than about 0.24 g/cm³ (15lbs/ft³). The apparent density of the main body element 1 can be determined by taking appropriate dimensional measurements from which the volume thereof is calculated, ascertaining its weight and then calculating the apparent density from the thusly obtained volume and weight values. Where the overall body element comprises a terminal flared end portion, the complexity of the dimensional measures, volume calculation and density calculation can be markedly reduced simply by cutting off said end portion prior to taking the dimensional and weight measurements of the main body element 1 referred to above.

The resilience of the viscoelastic polymeric foam material of construction of the body element 1 is sufficient as to ensure substantially complete recovery of the original shape and size of said body element when temporarily deformed and released in free space. In addition, in order that the benefit of alternative modes of insertion of the plug be realized, it is important that the polymeric foam employed for the construction of the body element 1 be viscoelastic in nature, in other words that it recover completely, but relatively slowly, when a deforming stress is removed therefrom. Such foams are said to exhibit viscoelastic, as opposed to elastic, behaviour. When such viscoelastic foams are employed in the construction of the main body element 1 there results an earplug construction which can be utilized either as a push-in or a roll-down type earplug. In the push-in mode, the main body element 1 composed of a viscoelastic polymeric foam can simply be forced into the ear canal by suitable manipulation of the stem 10. However, should the user desire it or should the ear canals of the user be exceptionally small or tortuous, thereby rendering the push-in mode of operation difficult, uncomfortable or undesirable, the user can then use an alternative method of insertion in a manner akin to that described by my U.S.Patent Re 29,487. Thus the viscoelastic foam main body element 1 of the invention can be simultaneously rolled and pressed axially between thumb and fingers so as to compress same to below the cross section of the ear canal into which it is to be inserted. Then, arising from the viscoelastic or slow recovery behaviour thereof, the user is afforded sufficient time to insert the recovering main body element 1 into the ear canal by suitable manipulation of the stem 10 before recovering of the body element 1 occurs to the extent of interference thereof with the enclosing ear canal wall.

A simple test for recovery rate is to provide a glass or clear plastic tube having an internal diameter 90% of the maximum diameter of the main body element 1 of the earplug under consideration. If the overall body element under consideration also includes a flared terminal end portion the flared terminal end portion is first cut off from the test specimens prior to testing of the remaining main body elements 1 thereof. The main body element 1 of the earplug is then rolled down tightly and lengthwise between thumb and fingers for about 30 seconds, released and immediately thereafter inserted into the tube by manipulation of the stem element. At the instant of release of the body element into the tube a stopwatch is started, the tube turned upright over a suitable support surface (such as a table top) and the tube cycled vertically close to and over the support surface at a cyclic rate of approximately 2/second and at an amplitude of approximately 6.35 mm (0.25 inch). The cycling is undertaking such that the free end 9 thereof remains in continuous contact with the support surface until such time as the recovering main body element 1 makes sufficient contact with the enclosed tube as to ride along with said tube during its vertical motion as opposed to sliding freely therewithin. The time for this to occur is noted and is taken as the 90% recovery time. The test procedure is carried out on at least three and preferably at least five sample earplugs of the candidate design and foam formulation in order to establish statistical significance and the results then averaged to provide an average 90% recovery time for the lot under test. Using this procedure the preferred viscoelastic polymeric foam main body element 1 of the present invention will have an average 90% recovery time of between about 10 to about 60 seconds.

There are many resilient polymeric foam compositions capable of meeting the viscoelastic or slow recovery property requirement of the invention. For instance, many of the externally and internally plasticized polymer foams disclosed in my U.S. Patent No. Re. 29,487 are generally suitable for use as a material of construction of the main body element 1. So, too, are many of the polyurethane foam compositions disclosed in U.S. Patent No. 4,158,087, to Louis Leonard Wood, June 12, 1979, entitled, "Urethane Foams Having Low Resiliency." The disclosure of each of the foregoing patents is incorporated herein, in its entirety, by reference.

Polyurethane foam compositions are generally preferred for the body element 1 due to their formulation flexibility, easy molding characteristics and economics. Of these, polyether polyurethane foams are even further preferred due to the generally soft surface "hand" or feel of resilient foam wares produced therewith. The polyether polyurethane foam compositions based on polyurethane prepolymers blended with acrylic latex modifiers in accordance with the above-mentioned Wood patent have been found to be useful in the practice of the invention. Such polyether polyurethane prepolymers are currently available from W. R. Grace Company under the "HYPOL" brand name. Suitable acrylic latex modifiers are available from Union Carbide Corporation under the "UCAR" brand name.

There follows an illustrative, non-limiting example.

### EXAMPLE

Ear plugs in accordance with Figure 1 hereof are produced by molding the foam body elements 1 thereof in a multi-cavity aluminum mold using a polyester polyurethane foam precursor composition containing a self-crosslinking, acrylic latex modifier. The mold comprises a closure plate having an aperture overlying the center of each body element 1 cavity and through which aperture there is received a stem element 10. Upon injection of the polyether polyurethane precursor charges into the respective mold cavities the cover is placed over the mold, thereby bringing the end 8 of each stem element captured in the mold cover apertures to a distance of 1.6 mm (0.054 inch) from the nose end of the mold cavity. The polyether polyurethane precursor charge in each cavity blows and partially cures in the closed cavity, the curing and blowing charge thereby filling the cavity, forming the body element 1 and recess 7 and, at the same time, bonding the end 8 of the stem 10 to the polyurethane material of the recess 7. The filling and curing of the mold cavities is undertaken at ambient temperature and, after 10 minutes in the closed mold, the closure plate is removed and the finished earplug wares removed, placed in a fabric bag and dried in a clothes dryer set at the "Medium" cycle in order to remove excess water from the formed wares and to complete the curing of the body elements 1. The particular polyether polyurethane foam precursor formulation employed was as follows:

| Ingredient | Parts by Weight |
|---|---|
| HYPOL 2002, polyether polyurethane prepolymer | 100 |
| UCAR 154, self cross-linking acrylic resin latex | 79.2 |
| Water containing 8.8 wt. percent PLURONIC F68 surfactant (BASF Wyandotte) | 19.8 |
| Sun Yellow YFD, a yellow colorant manufactured by Sun Chemical Company | 1.0 |
| The stem 10 elements were composed of a rolled paper rod stock having a uniform diameter of 4.0 mm (0.156 inch) and a length of 28.7 mm (1.130 inch). The resulting cured earplugs in accordance with Figure 1 hereof had the following dimensions: | |
| Length of body element 1 | 6.7 mm (0.262 inch) |
| Maximum diameter of body element 1, taken at base 6 | 13.0 mm (0.510 inch) |
| Exterior shape of body element 1 | hemispherical |

The density of the main foam body element 1 was determined by direct dimensional and weight measurement thereof. The main foam body element 1 was conditioned at room temperature and 50% relative humidity for at least about 24 hours. After carefully separating the stem 10 therefrom, the body element 1 was then weighed on an analytical balance capable of resolving 0.0001 gm. Dimensional measurements thereof were taken using an optical comparator or microscope at a magnification of at least 10 X. In the particular embodiment hereof, referring to Figure 1, the volume of the main foam body element 1 was calculated by first determining the overall volume of the hemispherical body and subtracting from this volume the calculated volume of the cylindrical recess 7. Using this protocol, the density of the main foam body element 1 was found to be 0.216 g/cm³ (13.45 lbs/ft³).

The foam main body element 1 was subjected to Shore 00 Durometer analysis and found to have a Durometer value of about 30.

The average 90% recovery rate of the earplugs was determined as disclosed hereinbefore, utilizing as a gauge a cylindrical glass tube having an internal diameter of 11.7 mm (0.459 inch). Said average 90% recovery rate was determined to be 15.4 seconds.

Several human subjects, having widely varying ear canal sizes and geometries, utilized the earplugs of this example in noisy industrial environments. All subjects reported that the earplugs were easy to use, whether by the push-in or roll-down modes of insertion, were comfortable throughout insertion, wearing and removal and provided adequate attenuation for their needs.

While I have described and shown certain present preferred embodiments of the invention, it is to be distinctly understood that the invention is not limited thereto, but may be variously embodied within the Scope of the following claims.

## Claims

1. A hearing protective earplug comprising:
a soft, resilient, polymeric foam body (100) comprising a smoothly contoured homogeneous viscoelastic main body element (1) free of lateral projections and internal cavities other than a stem receiving recess, having a rounded nose end (4) and a maximum cross sectional dimension along its length of 13.73 mm, said foam body (100) comprising a base end (6) extending linearly across the maximum dimension of said body element opposite said nose end (4) of said main body element (1);
an elongate stem (10) having two ends (8,9) one end (8) thereof being embedded in and secured to the interior of the nose end (4) portion of said main body element (1), said stem extending axially and rearwardly from said nose end portion (4) and through said base end (6), the other end (9) of said stem (10) terminating at a point exterior said base end (6), said stem (1) being sufficiently stiff to allow manipulation of said main body element into an ear canal whether in a precompressed or uncompressed condition; said viscoelastic main body element (1) of said earplug having a 90% recovery rate of between 2 and 120 second and capable of being inserted in an ear canal in either a precompressed or uncompressed condition.

2. The earplug of Claim 1 wherein said main body element has a 90% recovery rate of between 10 and 16 seconds.

3. The earplug of Claim 1 wherein the apparent density of said main body element is no greater than about 0.32 g/cm³.

4. The earplug of Claim 1 wherein the apparent density of said main body element is no greater than about 0.24 g/cm³.

5. The earplug of Claim 1 wherein the exterior shape of said main element (1) is hemispherical and the length to diameter ratio thereof is 0.5 to 1.

6. The earplug of Claim 1 wherein the exterior shape of said main body element is bullet shaped.

7. The earplug of Claim 1 wherein said foam body (10) is composed of a polyurethane.

8. The earplug of Claim 7 wherein said polyurethane is a polyether polyurethane.

9. The earplug of Claim 7 wherein said foam body is composed of a polyether polyurethane containing an acrylic latex modifier.

10. The earplug of Claim 1 wherein the maximum cross sectional dimension of that portion of said stem (10) element embedded in said main body element (1) is between 3.2 mm and 4.0 mm.

11. The earplug of Claim 7 wherein said polyurethane foam body is formed by molding of a polyurethane foam precursor formulation and wherein said stem element is secured thereinto during said molding.

12. The earplug of Claim 11 wherein said polyurethane foam precursor formulation contains an acrylic latex modifier.

13. The earplug of Claim 1 wherein the said main body element has an outboard end which transitions into a terminal flared end portion having a maximum cross sectional dimension which is sufficiently large to enable the said terminal flared end portion to function as a stop means upon insertion of the earplug into the ear canal.

14. The earplug of Claim 1 wherein said main body element has a circular cross section.

15. The earplug of Claim 1 wherein said main body element (1) has an ovoid cross section.

## Patentansprüche

1. Ein Gehörschutz-Ohrstöpsel mit
einem weichen, elastischen Polymer-Schaumkörper (100), der einen glatt umrissenen, homogenen, viskoelastischen Grundkörper (1), der außer einer stielaufnehmenden Vertiefung frei von seitlichen Vorsprüngen und inneren Hohlräumen ist, eine runde Nasenspitze (4) und eine maximale Abmessung in einem Querschnitt entlang seiner Länge von 13,73 mm hat, wobei dieser Schaumkörper (100) eine Basisstirnfläche aufweist, die sich gradlinig entlang der maximalen Abmessung dieses Grundkörpers und gegenüberliegend der Nasenspitze dieses Grundkörpers (1) erstreckt, und
einem länglichen Stiel (10) mit zwei Enden (8, 9), von denen ein Ende (8) im Innern des Bereiches der Nasenspitze (4) dieses Grundkörpers eingebettet und befestigt ist, wobei dieser Stiel sich in axialer Richtung und rückwärtig von diesem Nasenspitzenbereich (4) und durch die Basisstirnfläche (6) erstreckt, das andere Ende (9) dieses Stiels (10) an einem Punkt außerhalb der Basisstirnfläche (6) aufhört, wobei dieser Stiel (10) genügend steif ist, um eine Manipulation des Grundkörpers in den Gehörgang hinein entweder in einem vorkomprimierten oder unkomprimierten Zustand zuzulassen, wobei dieser viskoelastische Grundkörper (1) dieses Gehörstöpsels eine Regenerationsrate von 90 % zwischen 2 und 120 sec hat und in einen Gehörkanal entweder im vorkomprimierten oder unkomprimierten Zustand einführbar ist.

2. Ohrstöpsel nach Anspruch 1, bei dem der Grundkörper eine Regenerationsrate von 90 % zwischen 10 und 16 sec hat.

3. Ohrstöpsel nach Anspruch 1, bei dem die scheinbare Dichte des Grundkörpers nicht größer ist als 0,32 g/cm³.

4. Ohrstöpsel nach Anspruch 1, bei dem die scheinbare Dichte des Grundkörpers nicht größer ist als 0,24 g/cm³.

5. Ohrstöpsel nach Anspruch 1, bei dem die äußere Form des Grundkörpers (1) halbkugelig ist und dessen Verhältnis von Länge zu Durchmesser 0,5 bis 1 ist.

6. Ohrstöpsel nach Anspruch 1, bei dem die äußere Form des Grundkörpers geschoßförmig ist.

7. Ohrstöpsel nach Anspruch 1, bei dem der Schaumkörper (100) aus einem Polyurethan besteht.

8. Ohrstöpsel nach Anspruch 7, bei dem dieses Polyurethan ein Polyäther-Polyurethan ist.

9. Ohrstöpsel nach Anspruch 7, bei dem dieser Schaumkörper aus einem Polyäther-Polyurethan besteht, das einen Acryl-Latex-Modifikator beinhaltet.

10. Ohrstöpsel nach Anspruch 1, bei dem im Querschnitt die maximale Abmessung des Teiles von dem Stiel (10), der in den Grundkörper eingebettet ist, zwischen 3,2 mm und 4 mm liegt.

11. Ohrstöpsel nach Anspruch 7, bei dem der Polyurethanschaumkörper durch das Formen eines Polyurethanschaumvorläufers gebildet ist und bei dem das Stielelement während der Formgebung in diesem befestigt worden ist.

12. Ohrstöpsel nach Anspruch 11, bei dem der Polyurethanschaumvorläufer einen Acryl-Latex-Modifikator beinhaltet.

13. Ohrstöpsel nach Anspruch 1, bei dem der Grundkörper ein äußeres Ende hat, welches in einen sich nach außen erweiterenden Endbereich übergeht, mit einer maximalen Querschnittsabmessung, die genügend groß ist, um diesen begrenzenden sich nach außen erweiterenden Endbereich zu ermöglichen, bei der Einführung des Gehörstöpsels in den Gehörgang als Stopper zu fungieren.

14. Ohrstöpsel nach Anspruch 1, bei dem der Grundkörper einen kreisförmigen Querschnitt hat.

15. Ohrstöpsel nach Anspruch 1, bei dem der Grundkörper (1) einen eiförmigen Querschnitt hat.

## Revendications

1. Bouchon de protection auriculaire comprenant :
un corps en mousse polymère souple, élastique (100) comprenant un élément de corps principal (1) viscoélastique et homogène, à contour régulier, exempt de saillies latérales et de cavités internes autre qu'un évidement de réception de tige, ayant une extrémité de bout arrondie (4) et une dimension maximale en section transversale sur sa longueur de 13,73 mm, ledit corps en mousse (100) comprenant une extrémité de base (6) s'étendant linéairement sur la dimension maximale dudit élément de corps opposé à ladite extrémité de bout (4) dudit élément de corps principal (1) ;
une tige allongée (10) avec deux extrémités (8, 9), dont une extrémité est noyée dans et fixée à l'intérieur de la portion d'extrémité de bout (4) dudit élément de corps principal (1), ladite tige s'étendant axialement et vers l'arrière depuis ladite portion d'extrémité de bout (4) et à travers ladite extrémité de base (6), l'autre extrémité (9) de ladite tige (10) se terminant à un point extérieur à ladite extrémité de base (6), ladite tige (1) étant suffisamment rigide pour permettre la manipulation dudit élément de corps principal dans un canal auriculaire que ce soit à l'état précomprimé ou non comprimé ; ledit élément de corps principal viscoélastique (1) dudit bouchon auriculaire ayant un taux de récupération de 90% compris entre 2 et 120 secondes et apte à être inséré dans un canal auriculaire soit à l'état précomprimé soit à l'état non comprimé.

2. Bouchon auriculaire selon la revendication 1, où ledit élément de corps principal a un taux de récupération de 90% compris entre 10 et 16 secondes.

3. Bouchon auriculaire selon la revendication 1, où la densité apparente dudit élément de corps principal n'est pas supérieure à environ 0,32 g/cm³.

4. Bouchon auriculaire selon la revendication 1, où la densité apparente dudit élément de corps principal n'est pas supérieure à environ 0,24 g/cm³.

5. Bouchon auriculaire selon la revendication 1, où la forme extérieure dudit élément principal (1) est hémisphérique et le rapport longueur à largeur de celui-ci est de 0,5 à 1.

6. Bouchon auriculaire selon la revendication 1, où la forme extérieure dudit élément de corps principal est une forme de balle.

7. Bouchon auriculaire selon la revendication 1, où ledit corps en mousse (10) est constitué d'un polyuréthane.

8. Bouchon auriculaire selon la revendication 7, où ledit polyuréthane est un polyéther-polyuréthane.

9. Bouchon auriculaire selon la revendication 7,où ledit corps en mousse est constitué d'un polyéther-polyuréthane contenant un modificateur acrylique de latex.

10. Bouchon auriculaire selon la revendication 1, où la dimension maximale en section transversale de cette portion dudit élément de tige (10) noyé dans ledit élément de corps principal (1) est comprise entre 3,2 mm et 4,0 mm.

11. Bouchon auriculaire selon la revendication 7, où ledit corps en mousse de polyuréthane est formé par moulage d'une formulation de précurseur de mousse polyuréthane et où ledit élément de tige est fixé dans celui-ci pendant ledit moulage.

12. Bouchon auriculaire selon la revendication 11, où ladite formulation de précurseur de mousse de polyuréthane contient un modificateur acrylique de latex.

13. Bouchon auriculaire selon la revendication 1, où ledit élément de corps principal a une extrémité extérieure qui se termine par une portion d'extrémité évasée terminale ayant une dimension maximale en section transversale qui est suffisamment grande pour permettre que ladite portion d'extrémité évasée terminale fonctionne comme moyen d'arrêt lors de l'insertion du bouchon auriculaire dans le canal auriculaire.

14. Bouchon auriculaire selon la revendication 1, où ledit élément de corps principal a une section transversale circulaire.

15. Bouchon auriculaire selon la revendication 1, où ledit élément de corps principal (1) a une section transversale ovoïde.
